(19) Europäisches Patentamt
European Patent Office
Office européen des brevets

(11) Veröffentlichungsnummer: **0 283 817**
**B1**

# (12) EUROPÄISCHE PATENTSCHRIFT

(45) Veröffentlichungstag der Patentschrift: **27.12.90**

(21) Anmeldenummer: **88103509.1**

(22) Anmeldetag: **07.03.88**

(51) Int. Cl.[5]: **C08F 220/60,** C08F 220/34, A61K 7/11 // (C08F220/60, 220:04, 220:12, 220:60, 220:34),(C08F220/34, 220:04, 220:12, 220:60, 220:34)

## (54) Zwitterionische Polymere und deren Verwendung in Haarbehandlungsmitteln.

(30) Priorität: **16.03.87 DE 3708451**

(43) Veröffentlichungstag der Anmeldung: **28.09.88 Patentblatt 88/39**

(45) Bekanntmachung des Hinweises auf die Patenterteilung: **27.12.90 Patentblatt 90/52**

(84) Benannte Vertragsstaaten: **AT BE CH DE ES FR GB GR IT LI LU NL SE**

(56) Entgegenhaltungen:
**EP-A- 0 082 657**
**DE-A- 2 817 369**
**FR-A- 2 110 268**
**FR-A- 2 393 011**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Schieferstein, Ludwig, Dr., Am Hang 15, D-4030 Ratingen 1(DE)**
Erfinder: **Höffkes, Horst, Dr., Carlo Schmid-Str. 113, D-4000 Düsseldorf(DE)**
Erfinder: **Seidel, Kurt, Nosthoffenstr. 59, D-4000 Düsseldorf 13(DE)**
Erfinder: **Giede, Karl, Schlehenweg 12, D-4010 Hilden(DE)**
Erfinder: **Busch, Peter, Dr., Gottfried August Bürger-Str. 10, D-4006 Erkrath(DE)**

EP 0 283 817 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue wasserlösliche, zwitterionische Polymerisate mit guter Verträglichkeit mit anionischen und kationischen Tensiden und guten haaravivierenden und frisurstabilisierenden Eigenschaften und deren Verwendung in Haarbehandlungsmitteln, insbesondere in Haarshampoos.

Zwitterionische Polymere zeichnen sich dadurch aus, daß sie anionische Gruppen, meist Carboxylgruppen und quartäre Ammoniumgruppen im Molekül enthalten. Es sind zahlreiche zwitterionische Polymerisate bekannt. Es ist auch bekannt, zwitterionische Polymerisate in Haarbehandlungsmitteln zu verwenden. DE-OS 21 50 557 beschreibt die Verwendung von Polymerisaten zwitterionischer Monomerer in Haarfestlegemitteln. Auch aus DE-OS 28 17 369 war die Verwendung von Copolymerisaten aus Dimethylaminoethylmethacrylat, Acrylsäure und Methylmethacrylat in Haarfestlegemitteln und Shampoos bekannt. Zwitterionische Copolymere aus Monomeren mit quartären Ammoniumgruppen und Monomeren mit Carboxylgruppen sind aus EP-A 82 657 bekannt, wo solche Polymerisate als Wasserstein- und Korrosionsinhibitoren empfohlen werden.

Die für Haarbehandlungs- und Haarfestlegemitteln bekannten zwitterionischen Polymeren zeigen, insbesondere in Formulierungen mit anionischen Tensiden, den Nachteil, daß die haaravivierenden und haarfestigenden Eigenschaften im Verlaufe längerer Lagerzeit allmählich verlorengehen. Rein kationische wasserlösliche Polymere haben den Nachteil, daß die haarfestigenden und frisurstabilisierenden Eigenschaften zu wenig ausgeprägt sind. Es bestand daher die Aufgabe, haarfestigende und haaravivierende Komponenten für Haarbehandlungsmittel, insbesondere für Mittel zur Pflege und Reinigung des Haars mit einem Gehalt an anionischen Tensiden zu finden, die ihre Wirkung auch über längere Lagerzeiten in der Formulierung beibehalten.

Es wurde gefunden, daß die gestellten Aufgaben in hohem Maße erfüllt werden durch zwitterionische Polymerisate, die sich im wesentlichen zusammensetzen aus

(A) 30 bis 70 Mol% Monomeren mit quartären Ammoniumgruppen der Formel (I)

(I) $R^1\text{-}CH{=}CR^2\text{-}CO\text{-}Z\text{-}(C_nH_{2n})\text{-}N^{(+)}(CH_3)_3A^{(-)}$

in der $R^1$ und $R^2$ Wasserstoff oder Methylgruppen sind, Z ein Sauerstoffatom oder eine -NH-Gruppe, n = 2 bis 5 und $A^{(-)}$ein Chlorid-, Bromid-, Methoxysulfat- oder Ethoxysulfat-Anion ist,

(B) 10 bis 30 Mol% monomeren Carbonsäuren der Formel (II)

(II) $R^3\text{-}CH{=}CR^4\text{-}COOH$

in der $R^3$ und $R^4$ Wasserstoff oder Methylgruppen sind,

(C) 10 bis 30 Mol% monomeren Estern der Formel (III)

(III) $R^5\text{-}CH{=}CR^6\text{-}COOR^7$

in der $R^5$ und $R^6$ Wasserstoff oder Methylgruppen sind und $R^7$ eine Methyl- oder Ethylgruppe ist und

(D) 0 bis 40 Mol% Monomeren mit tertiären Aminogruppen der Formel (IV)

(IV) $R^8\text{-}CH{=}CR^9\text{-}CO\text{-}Z\text{-}(C_nH_{2n})\text{-}NR^{10}R^{11}$

in der $R^8$ und $R^9$ Wasserstoff oder Methylgruppen sind und $R^{10}$ und $R^{11}$ Wasserstoff oder Alkylgruppen mit 1 bis 4 C-Atomen sind oder gemeinsam mit dem Stickstoffatom einen Piperidin-, Piperazin-, Pyrrolidin- oder Morpholin-Ring bilden, Z ein Sauerstoffatom oder eine -NH-Gruppe und n = 2 bis 5 ist.

Die erfindungsgemäßen zwitterionischen Polymerisate sind in Wasser und in wäßrigen Lösungen anionischer, kationischer, ampholytischer, zwitterionischer und nichtionogener Tenside gut löslich und behalten in wäßrigen Lösungen anionischer Tenside ihre guten, haaravivierenden und haarfestigenden Eigenschaften auch über längere Lagerzeiten bei. Aufgrund der genannten Eigenschaften eignen sich die erfindungsgemäßen zwitterionischen Polymerisate als festigende und haaravivierende Komponente in wäßrigen Zubereitungen zur Reinigung und Pflege der Haare. Solche Zubereitungen können z. B. Haarshampoos, Haarnachspülmittel, Haarfestiger, Haar-Fönwellmittel sowie wäßrige Färbemittel, Dauerwellmittel oder Dauerwell-Fixiermittel sein.

Die erfindungsgemäßen zwitterionischen Polymeren werden aus Monomeren der Formeln (I), (II), (III) und gegebenenfalls (IV) nach an sich bekannten Polymerisationsverfahren in wäßrigalkoholischer Lösung hergestellt. Als Starter wird eine radikalbildende Substanz wie z. B. Kalium- oder Ammoniumperoxidsulfat, tert.-Butylhydroperoxid, Azobis-(cyanpentansäure) oder bevorzugt Azoisobutyronitril in geringen Mengen zugegeben. Die Herstellung von zwei erfindungsgemäßen Copolymerisaten wird als Beispiel wiedergegeben.

Als Monomere der Formel (I) können z. B. Derivate der Acrylsäure, Methacrylsäure-, Crotonsäure- oder 2-Methylcrotonsäure eingesetzt werden. Besonders geeignete Monomere mit quartären Ammoniumgruppen sind z. B. Methacryloxyethyl-trimethyl-ammonium-methosulfat oder Methacrylamidopropyltrimethyl-ammonium-chlorid.

Als monomere Carbonsäuren der Formel (II) eignen sich Acrylsäure, Methacrylsäure, Crotonsäure und 2-Methyl-crotonsäure. Bevorzugt werden Acryl- oder Methacrylsäure eingesetzt.

Als monomere Ester der Formel (III) können die Methyl- und Ethylester von Acrylsäure, Methacrylsäure, Crotonsäure und 2-Methylcrotonsäure verwendet werden. Bevorzugt geeignet sind die Methylester der Acryl- und/oder Methacrylsäure.

Als Monomere mit tertiären Aminogruppen der Formel (IV) eignen sich bevorzugt Acryl- und Methacrylsäurederivate, wie z. B. Dimethylaminoethyl-methacrylat, Dimethylaminopropylmethacrylamid, 2-tert.-Butylaminoethylmethacrylat oder Dimethylamino-neopentylacrylat.

Bevorzugte zwitterionische Polymere setzen sich im wesentlichen zusammen aus

(a) 30 bis 70 Mol% Methacrylamidopropyl-trimethylammoniumchlorid (MAPTAC),
(B) 10 bis 30 Mol% Acrylsäure (AS) oder Methacrylsäure (MAS),
(C) 10 bis 30 Mol% Methylacrylat (MA) oder Methylmethacrylat (MMA) und
(D) 0 bis 40 Mol% Dimethylaminoethyl-methacrylat (DMAEMA).

Durch den Anteil der Komponente (D), d. h. von Monomeren der Formel (IV) lassen sich die Eigenschaften der zwitterionischen Polymeren in Richtung auf eine verbesserte Avivagewirkung modifizieren. Zwitterionische Polymere mit einem besonders hohen Anteil an Komponente (A), d. h. von Monomeren der Formel (I) weisen dagegen eine besonders ausgeprägte festigende, frisurstabilisierende Wirkung auf.

Besonders bevorzugt ist die Verwendung der erfindungsgemäßen zwitterionischen Polymeren in wäßrigen Zubereitungen mit einem Gehalt an anionischen Tensiden. Eine bevorzugte Ausführungsform der Erfindung ist daher ein Haarshampoo auf wäßriger Basis, dadurch gekennzeichnet, daß es 0,1 bis 10 Gew.-% eines erfindungsgemäßen zwitterionischen Polymers und 5 bis 25 Gew.-% eines anionischen Tensids enthält.

Als anionische Tenside eignen sich in erfindungsgemäßen Haarbehandlungsmitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind die Natrium-, Kalium-, Ammonium-, Mono-, Di- und Trialkanolammoniumsalze mit 2 oder 3 C-Atomen in der Alkanolgruppe, von

- linearen Fettsäuren mit 10 bis 22 C-Atomen (Seifen),
- Ethercarbonsäuren der Formel $R^1\text{-}O\text{-}(CH_2\text{-}CH_2O)_x\text{-}CH_2\text{-}COOH$ in der $R^1$ eine lineare Alkylgruppe mit 10 bis 22 C-Atomen und x = 0 oder 1 bis 10 ist,
- Acylsarcosinen mit 10 bis 18 C-Atomen in der Acylgruppe,
- Acyltauriden mit 10 bis 18 C-Atomen in der Acylgruppe,
- Acylisethionaten mit 10 bis 18 C-Atomen in der Acylgruppe,
- Sulfobernsteinsäuremono- und dialkylestern mit 8 bis 18 C-Atomen in der Alkylgruppe und Sulfobernsteinsäuremonoalkylpolyoxyethylestern mit 8 bis 18 C-Atomen in der Alkylgruppe und 1 bis 6 Oxyethylgruppen,
- linearen Alkansulfonaten mit 12 bis 18 C-Atomen,
- linearen Alpha-Olefinsulfonaten mit 12 bis 18 C-Atomen,
- Alpha-Sulfofettsäuremethylestern von Fettsäuren mit 12 bis 18 C-Atomen,
- Alkylsulfaten und Alkylpolyglykolethersulfaten der Formel $R^1\text{-}(OCH_2\text{-}CH_2)_z\text{-}OSO_3^{(-)}M^{(+)}$, in der $R^1$ eine bevorzugt lineare Alkylgruppe mit 10 bis 18 C-Atomen und z = 0 oder eine Zahl von 1 bis 10 und $M^{(+)}$ ein Lithium-, Natrium-, Kalium-, Magnesium-, Ammonium-, Mono-, Di-, oder Trialkanolammonion mit 2 oder 3 C-atomen der Alkynolgruppe ist.

Bevorzugt geeignet sind Alkylsulfate- und Alkylpolyglykolethersulfate mit 10 bis 18 C-Atomen in der Alkylgruppe und bis zu 12 Glykolethergruppen im Molekül.

Neben den genannten anionischen Tensiden können die erfindungsgemäßen Haarshampooformulierungen alle für diesen Zweck bekannten Hilfs- und Zusatzmittel in den dafür üblichen Mengen enthalten. Dies sind insbesondere nichtionogene, amphotere und zwitterionische Tenside.

Nichtionische Tenside sind vor allem die Anlagerungsprodukte von 2 bis 20 Mol Ethylenoxid an bevorzugt lineare Alkohole mit 12 bis 18 C-Atomen, an Alkylphenole mit 8 bis 15 C-Atomen in der Alkylgruppe, an Fettsäuren mit 12 bis 18 C-Atomen, an Fettsäurepartialglyceride, an Fettsäure-sorbitan-partialester, an Fettsäurealkanolamide und an Methylglucosid-Fettsäureester. Weitere geeignete nichtionogene Tenside sind Alkyl(oligo)-glucoside, Alkylaminoxid-Tenside und Fettsäurealkanolamide. Amphotere Tenside sind z. B. die Alkyl($C_8$-$C_{18}$)-trimethylammonio-glycinat oder Acyl($C_8$-$C_{18}$)-aminopropyltrimethylammonio-glycinat.

Auch bestimmte, aniontensid-verträgliche kationische Tenside können in den erfindungsgemäßen Haarshampoos enthalten sein. Solche kationischen Tenside sind z. B. aus DE-OS 34 42 175 bekannt.

Die erfindungsgemäßen wäßrigen Zubereitungen zur Reinigung und Pflege der Haare können neben den obligatorischen zwitterionischen Polymeren alle für den jeweiligen Anwendungszweck üblichen Hilfs- und Zusatzmittel enthalten.

Für Haarnachspülmittel sind dies z. B. kationische Tenside, insbesondere oberflächenaktive quartäre Ammoniumsalze, Fettalkohole mit 12 bis 22 C-Atomen, Fettsäurepartialglyceride, kosmetische Öl- und Fettkomponente und wasserlösliche Polymere mit verdickender Wirkung. Für Haarfestiger und Fönwellmittel sind dies z. B. ebenfalls kationische Tenside, kationische, nichtionogene oder anionische Polymere und niedere Alkohole. Haarfärbemittel enthalten direktziehende Farbstoffe oder Oxidationsfarbstoffvorprodukte, anionische oder nichtionogene Tenside, Ammoniak oder Alkanolamine sowie ggf. Antioxidantien, Dauerwellfixiermittel enthaltend ein Oxidationsmittel, z. B. $H_2O_2$, $H_2O_2$-Anlagerungsverbindungen oder z. B. Kaliumbromat sowie anionische oder nichtionogene Tenside.

Die erfindungsgemäßen zwitterionischen Polymerisate sind in den genannten wäßrigen Zubereitungen zur Pflege und Reinigung der Haare bevorzugt in einer Menge von 0,1 bis 10 Gew.-%, insbesondere von 0,1 bis 2 Gew.-% enthalten.

Die folgenden Beispiele sollen den Erfindungsgegenstand näher erläutern, ohne ihn hierauf zu beschränken.

## <u>Beispiele</u>

1. <u>Herstellung eines Copolymerisats aus 3 Mol MAPTAC, 1 Mol Acrylsäure und 1 Mol Methylmethacrylat</u>

In einem Reaktor mit Impellerrührer, Heizung, Kühlung, Rückfluß- und Vorlaufkühlung sowie Temperaturmessung wurden
2,4 kg Methylmethacrylat (MMA)
16,0 kg Isopropanol
vorgelegt und darin
0,1 kg Azoisobutyronitril
unter Rühren aufgelöst. Dann wurden
31 kg einer 50 Gew.-%igen wäßrigen Lösung von Methacrylamidopropyl-trimethylammoniumchlorid (MAPTAC)
1,7 kg Acrylsäure (AS) und
47,2 kg Wasser
zugesetzt. Der Ansatz wurde mit ca.
1,6 kg Ammoniaklösung (25 Gew.-% in Wasser)
auf den pH-Wert = 7,0 eingestellt.

Dann wurde bei einer Manteltemperatur von 65 °C 30 Minuten gerührt, wobei eine leichte Erwärmung stattfand. Danach wurde auf 80 °C erwärmt und weitere 2 Stunden gerührt. Nach beendeter Reaktion wurden 20 kg Wasser zugesetzt und diese Menge unter Normaldruck (1 bar) als Azeotyp wieder abdestilliert. Nach Abkühlung auf 30 °C zeigte die Polymerlösung folgende Kennwerte:
pH-Wert:6,8
Getrocknete Acetonfällung:125 % d. Th.
Spezifische Viskosität: (1 %ig in 1 n $NaNO_3$-Lösung)0,65

2. <u>Herstellung eines Copolymerisats aus 2 Mol MAPTAC, 1 Mol Acrylsäure und 1 Mol Methylmethacrylat und 2 Mol DMAEMA</u>

In einem Reaktor mit Impellerrührer, Heizung, Kühlung, Rückfluß- und Vorlaufkühlung sowie Temperaturmessung wurden
2,1 kg Methylmethacrylat
16,0 kg Isopropanol
vorgelegt und
0,1 kg Azoisobutyronitril
unter Rühren aufgelöst. Dann wurden
18,3 kg einer 50 Gew.-%igen wäßrigen Lösung von Methacrylamidopropyl-trimethylammoniumchlorid (MAPTAC)
6,5 kg Dimethylaminoethyl-methacrylat (DMAEMA) und
52,9 kg Wasser
zugesetzt. Nach vorsichtiger Zugabe von
1,5 kg Acrylsäure
wurde der Ansatz mit ca. 2,6 kg verd. Schwefelsäure (30 Gew.-% in Wasser) auf den pH-Wert = 7,0 eingestellt und auf 65 °C erwärmt. Nach 30 Minuten Rühren bei dieser Temperatur, wobei eine leichte Erwärmung stattfand, wurde auf 80 °C erwärmt und weitere 2 Stunden gerührt. Danach wurden 20 kg Wasser zugegeben und diese Menge unter Normaldruck (1 bar) als Azeotrop wieder abdestilliert. Nach Abkühlung auf 30 °C zeigte die Polymerlösung folgende Kennwerte:
pH-Wert:6,9
Getrocknete Acetonfällung:145 % d. Th.
Spezifische Viskosität: (1 %ig in 1 n $NaNO_3$-Lösung)0,65

| Anwendungsbeispiele | |
|---|---|
| 3. Haarshampoo | |
| | Gew.–% |
| Fettalkohol ($C_{12-14}$) poly(3 EO)glykolether-sulfat, Na-Salz, 28%ige wäßrige Lösung | 30 |
| Kokosacy($C_{12}$–$C_{18}$)-aminopropyl-dimethylglycin, 30%ige wäßrige Lösung | 20 |
| Copolymer-Lösung Beispiel 1 (ca. 20 %ig) | 5 |
| Wasser | ad 100 |

| 4. Haarspülung | |
|---|---|
| | Gew.–% |
| Fettalkohol($C_{12-14}$)poly(3 EO)glykolether-sulfat, Na-Salz, 28%ige wäßrige Lösung | 5 |
| Copolymer-Lösung Beispiel 2 (ca. 20 %ig) | 2 |
| Wasser | ad 100 |

| 5. Haarspülung | |
|---|---|
| | Gew.-% |
| N-(2-Hydroxyhexadecyl-1)-N,N-dimethyl-N-(2-hydroxyethyl-ammoniumchlorid, 28%ige wäßrige Lösung | 4 |
| Copolymer-Lösung Beispiel 2 (ca. 20%ig) | 5 |
| Wasser | ad 100 |

| 6. Fönwellmittel | |
|---|---|
| | Gew.-% |
| N-2-Hydroxyhexadecyl-1)-N,N-dimethyl-N-(2-hydroxyethyl)-ammoniumchlorid, 28 %ige wäßrige Lösung | 0.2 |
| Isopropanol | 15,0 |
| Copolymer-Lösung Beispiel (ca. 20%ig) | 6,0 |
| Wasser | ad 100 |

| 7. Haarfestiger | |
|---|---|
| | Gew.-% |
| Polyvinylpyrrolidon-Polyvinylacetat-Copolymer (60 : 40) | 1,4 |
| Copolymer-Lösung Beispiel 1 (ca. 20%ig) | 4,0 |
| Ethanol | 20,0 |
| Wasser | ad 100 |

8. Haarfärbemittel

| | Gew.-% |
|---|---|
| Talgfettalkohol($C_{16-18}$) | 8,0 |
| Fettalkohol(C12–14)poly(3EO)glykolethersulfat, Na-Salz, 28 %ige wäßrige Lösung | 20,0 |
| Copolymer-Lösung Beispiel 1 (ca. 20 %ig) | 2,5 |
| Oxidationsfarbstoff-Vorprodukte | 2,0 |
| Wasser | ad 100 |

9. Dauerwellfixierungsmittel

| | | Gew.-% |
|---|---|---|
| Kaliumbromat | | 5,0 |
| Fettalkohol($C_{12-14}$)poly(3 EO)glykolethersulfat, Na-Salz, 28 %ige wäßrige Lösung | | 5,0 |
| Citronensäure | bis pH = | 4 |
| Copolymer-Lösung Beispiel 2 (ca. 20 %ig) | | 1,5 |
| Wasser | | ad 100 |

**Patentansprüche**

1. Zwitterionische Polymerisate, die sich im wesentlichen zusammensetzen aus
(A) 30 bis 70 Mol% Monomeren mit quartären Ammoniumgruppen der Formel (I)

(I)$R^1$-CH=C$R^2$-CO-Z-($C_nH_{2n}$)-$N^{(+)}$($CH_3$)$_3$$A^{(-)}$

in der $R^1$ und $R^2$ Wasserstoff oder Methylgruppen sind, Z ein Sauerstoffatom oder eine -NH-Gruppe, n = 2 bis 5 und $A^{(-)}$ein Chlorid-, Bromid-, Methoxysulfat- oder Ethoxysulfat-Anion ist,
(B) 10 bis 30 Mol% monomeren Carbonsäuren der Formel (II)

(II)$R^3$-CH=C$R^4$-COOH

in der $R^3$ und $R^4$ Wasserstoff oder Methylgruppen sind,
(C) 10 bis 30 Mol% monomeren Estern der Formel (III)

(III)$R^5$-CH=C$R^6$-COO$R^7$

in der $R^5$ und $R^6$ Wasserstoff oder Methylgruppen sind und $R^7$ eine Methyl- oder Ethylgruppe ist und
(D) 0 bis 40 Mol% Monomeren mit tertiären Aminogruppen der Formel (IV)

(IV)$R^8$-CH=C$R^9$-CO-Z-($C_nH_{2n}$)-N$R^{10}R^{11}$

in der $R^8$ und $R^9$ Wasserstoff oder Methylgruppen sind und $R^{10}$ und $R^{11}$ Wasserstoff oder Alkylgruppen mit 1 bis 4 C-Atomen sind oder gemeinsam mit dem Stickstoffatom einen Piperidin-, Piperazin-, Pyrrolidin- oder Morpholin-Ring bilden, Z ein Sauerstoffatom oder eine -NH-Gruppe und n = 2 bis 5 ist.

2. Zwitterionische Polymerisate gemäß Anspruch 1, dadurch gekennzeichnet, daß sie sich im wesentlichen zusammensetzen aus
(A) 30 bis 70 Mol% Methacrylamidopropyl-trimethylammoniumchlorid (MAPTAC),
(B) 10 bis 30 Mol% Acrylsäure (AS) oder Methacrylsäure (MAS),
(C) 10 bis 30 Mol% Methylacrylat (MA) oder Methylmethacrylat (MMA) und
(D) 0 bis 40 Mol% Dimethylaminoethyl-methacrylat (DMAEMA).

3. Verwendung von zwitterionischen Polymeren gemäß den Ansprüchen 1 oder 2 als festigende und haaravivierende Komponente in wäßrigen Zubereitungen zur Reinigung und Pflege der Haare.

4. Haarshampoo auf wäßriger Basis, dadurch gekennzeichnet, daß es 0,1 bis 10 Gew.-% eines zwitterionischen Polymers gemäß den Ansprüchen 1 oder 2 und 5 bis 25 Gew.-% eines anionischen Tensids enthält.

5. Haarshampoos gemäß Anspruch 4, dadurch gekennzeichnet, daß das anionische Tensid ein Alkylsulfat- oder Alkyl-polyglykolethersulfat der Formel (V)

(V) $R^1$-$(OCH_2CH_2)_z$-$OSO_3^{(-)}M^{(+)}$

ist, worin $R^1$ eine lineare Alkylgruppe mit 10 bis 18 C-Atomen, z = 0 oder eine Zahl von 1 bis 10 und $M^{(+)}$ein Lithium-, Natrium-, Kalium-, Magnesium-, Ammonium-, Mono-, Di- oder Trialkanolammoniumion mit 2 oder 3 C-Atomen in der Alkanolgruppe ist.

**Claims**

1. Zwitterionic polymers essentially consisting of (A) 30 to 70 mol-% monomers containing quaternary ammonium groups and corresponding to the following formula

(I) $R^1$–CH=$CR^2$–CO–Z–$(C_nH_{2n})$-$N^{(+)}(CH_3)_3$
$A^{(-)}$

in which $R^1$ and $R^2$ are hydrogen or methyl groups, Z is an oxygen atom or an -NH-group, n= 2 to 5 and $A^{(-)}$ is a chloride, bromide, methoxysulfate or ethoxysulfate anion, (B) 10 to 30 mol-% monomeric carboxylic acids corresponding to the following formula

(II) $R^3$–CH=$CR^4$–COOH

in which $R^3$ and $R^4$ are hydrogen or methyl groups, (C) 10 to 30 mol-% monomeric esters corresponding to the following formula

(III) $R^5$–CH=$CR^6$–$COOR^7$

in which $R^5$ and $R^6$ are hydrogen or methyl groups and $R^7$ is a methyl or ethyl group and (D) o to 40 mol-% monomers containing tertiary amino groups and corresponding to the following formula

(IV) $R^8$–CH=$CR^9$–CO–Z–$(C_nH_{2n})$–$NR^{10}R^{11}$

in which $R^8$ and $R^9$ are hydrogen or methyl groups and $R^{10}$ and $R^{11}$ are hydrogen or $C_{1-4}$ alkyl groups or, together with the nitrogen atom, form a piperidine, piperazine, pyrrolidine or morpholine ring, Z is an oxygen atom or an -NH-group and n= 2 to 5.

2. Zwitterionic polymers as claimed in claim 1, characterized in that they essentially consist of
(A) 30 to 70 mol-% methacrylamidopropyl trimethyl ammonium chloride (MAPTAC),
(B) 10 to 30 mol-% acrylic acid (AA) or methacrylic acid (MAA),
(C) 10 to 30 mol-% methyl acrylate (MA) or methyl methacrylate (MMA) and
(D) 0 to 40 mol-% dimethylaminoethyl methacrylate (DMAEMA).

3. The use of zwitterionic polymers as claimed in claim 1 or 2 as setting and hair-softening component in aqueous hair-washing and hair-care preparations.

4. A water-based hair shampoo, characterized in that it contains from 0.1 to 10% by weight of a zwitterionic polymer of the type claimed in claim 1 or 2 and from 5 to 25% by weight of an anionic surfactant.

5. Hair shampoos as claimed in claim 4, characterized in that the anionic surfactant is an alkyl sulfate or alkyl polyglycol ether sulfate corresponding to the following formula

(V) $R^1$–$(OCH_2CH_2)z$–$OSO_3$^ (–) $M^{(+)}$

in which $R^1$ is a linear $C_{10-18}$ alkyl group, z = o or a number of from 1 to 10 and $M^{(+)}$ is a lithium, sodium, potassium, magnesium, ammonium, mono-, di- or trialkanolammonium ion containing 2 or 3 carbon atoms in the alkanol group.

**Revendications**

1. Polymérisats zwittérioniques qui se composent essentiellement de (A) 30 à 70% mol de monomères ayant des groupes ammonium quaternaire de formule (I)

(I) $R_1$-CH=$CR_2$CO-Z-$(C_nH_{2n})$-$N^{(+)}(CH_3)_3$
$A^{(-)}$

dans laquelle $R_1$ et $R_2$ sont de l'hydrogène ou un radical méthyle Z est un atome d'oxygène ou un groupe -NH-, n= 2 à 5 et A $^{(-)}$ est un chlorure, un bromure, un anion méthoxylsulfate ou éthoxysulfate (B) 10 à 30% mol d'acides carboxyliques monomères de formule II

(II) $R_3$-CH=$CR_4$-COOH

dans laquelle $R_3$ et $R_4$ sont de l'hydrogène ou un radical méthyle (C) 10 à 30% mol d'esters monomériques de formule (III)

(III) $R_5$-CH=C$R_6$-COO$R_7$

dans laquelle $R_5$ et $R_6$ sont de l'hydrogène ou un radical méthyle et $R_7$ est un radical méthyle ou éthyle et (D) 0 à 40% mol de monomères ayant des groupes amine tertiaire de formule (IV)

(IV) $R_8$-CH=C$R_9$-CO-Z-($C_nH_{2n}$)=N$R_{10}R_{11}$

dans laquelle $R_8$ et $R_9$ sont de l'hydrogène ou un radical méthyle et $R_{10}$ et $R_{11}$ sont de l'hydrogène ou un radical alcoyle ayant de 1 à 4 atomes de carbone en ensemble forment avec l'atome d'azote un cycle piperidine, piperazine, pyrrolidine ou morpholine, Z est un atome d'oxygène ou un groupe -NH- et est égal à 2 à 5.

2. Polymérisats zwittérioniques selon la revendication 1 caractérisés par le fait qu'ils se composent essentiellement de
(A) 30 à 70% Mol de chlorure de (méthacrylamidopropyl) triméthylarmmonium (MAPTAC)
(B) 10 à 30% Mol d'acide Acrylique (AS) ou d'acide Méthacrylique (MAS)
(C) 10 à 30% Mol d'acrylate de méthyle (MA) ou de Méthacrylate de Méthyle (MMA) et
(D) o à 40% Mol de méthacrylate de diméthylamino éthyle (DMAEMA).

3. Utilisation de polymères zwittérioniques selon les revendications 1 ou 2, comme composants pour fortifier ou raviver les cheveux dans des compositions aqueuses en vue du nettoyage et du soin des cheveux.

4. Shampooing pour les cheveux à base aqueuse, caractérisé en ce qu'il contient de 0,1 à 10% en poids d'un polymère zwittérionique selon les revendications 1 ou 2 et de 5 à 25% en poids d'un agent tensio-actif anionique.

5. Shampooing pour les cheveux selon la revendication 4, caractérisé en ce que l'agent tensioactif anionique est un alcoyle sulfate ou un sulfate d'alcoyl-polyéther de glycol de formule (V)

(V) $R_1$-(OC$H_2$CH2)Z-OS$O_3$ $^{(-)}M^{(+)}$

dans laquelle $R_1$ est un radical alcoyle linéaire ayant de 10 à 18 atomes de carbone, Z = 0 ou un nombre de 1 à 10 et M est un ion lithium, sodium, Potassium, Magnesium, Ammonium, Mono-, Di- ou Trialcanol ammonium avec 2 ou 3 atomes de carbone dans le groupe alcanol.